# EUROPEAN PATENT APPLICATION

(11) **EP 1 508 329 A1**
(43) Date of publication of application: **23.02.2005**
(21) Application number: 04019682.6
(22) Date of filing: 19.08.2004
(51) Int. Cl.: A61K 9/00, A61K 9/14

(54) **Composition for an antifungal agent for suppressing fungal growth in an oral cavity**

(30) Priority: 20.08.2003 JP 2003296627
(71) Applicant: Matsumoto Dental University, Nagano 399-0781 (JP)
(72) Inventor: Wang, Pao-Li, Shiojiri Nagano 399-0781 (JP); Ito, Michio, Shiojiri Nagano 399-0781 (JP); Igarashi, Yoshimasa, Shiojiri Nagano 399-0781 (JP)
(74) Representative: VOSSIUS & PARTNER

(57) **Abstract**

A composition for an antifungal agent contains miconazole, which is an imidazole antifungal agent, and adhesive protein and is mainly used as a drug for treating denture candidasis. The adhesive protein is one selected from collagen, laminin and fibronectin.

## Description

This application claims priority to prior Japanese application JP 2003-296627, the disclosure of which is incorporated herein by reference.

This invention relates to a composition for an antifungal agent containing miconazole which is an imidazole antifungal agent.

An imidazole antifungal agent is a medicament having an imidazole group in a chemical structure. As the imidazole angifungal agent, miconazole (for example, miconazole nitrate) is known.

Miconazole exerts an antimycotic or antifungal effect mainly by inhibiting synthesis or formation of cell membranes of fungi.

Miconazole exhibits a strong bacteriocidal activity against various bacterial species, such as trichophyton, candida, microsporum, and epidermophyton, and aspergillus.

Further, miconazole also has a strong bacteriocidal activity against gram-positive bacteria, such as staphylococcus aureus and streptococcus phygenes.

In the fields of dental surgery and oral surgery, it is known that a dental prosthesis in an oral cavity tends to provide a medium for growth of candida. Therefore, a patient wearing a dental prosthesis often complains about symptoms of candidasis.

Thus, the dental prosthesis tends to cause the candidasis in the oral cavity. It is therefore important to take good care of the oral cavity, to appropriately prevent the candidasis in the oral cavity, and to establish a treatment method for the candidasis.

As a drug for treating denture candidasis, miconazole in the form of gel, i.e., miconazole gel is used. It is known that the miconazole gel is effective against candida albicans in the oral cavity (for example, see Japanese Patent Application Publication (JP-A) No. 9-110690).

However, it is also known that the miconazole gel has adverse effects caused by administration into the oral cavity in high concentration. As the adverse effects of the miconazole gel, many patients suffer from unpleasant symptoms, such as oral erosion and ulcer pain.

Therefore, the amount of the miconazole gel to be administered is generally limited. For example, in case of an adult, 200-400 mg miconazole (10-20 g miconazole gel) is daily applied in the oral cavity in four separate doses.

It is an object of this invention to provide a composition for an antifungal agent, which enables miconazole to be adhered to and held on a dental prosthesis for a long time so as to increase a fungal activity suppression ratio and to suppress fungal growth.

It is another object of this invention to provide a composition for an antifungal agent, which exhibits an excellent effect at a lower concentration than that of an existing antifungal agent containing miconazole without adverse effects.

According to this invention, there is provided a composition for an antifungal agent, comprising miconazole, which is an imidazole antifungal agent, and adhesive protein.
Fig. 1 is a graph showing fungal activity suppression ratios in case where a composition according to this invention and a comparative composition were used, respectively; and
Fig. 2 is a graph showing adhesion ratios of a composition according to this invention and a comparative composition with respect to a dental prosthesis.

Now, a composition for an antifungal agent according to this invention will be described. The composition for an antifungal agent comprises miconazole, which is an imidazole antifungal agent, and adhesive protein. As the adhesive protein, collagen is used. The collagen is processed into powder to be used as collagen powder. Thus, the composition for an antifungal agent is a mixture of miconazole and the collagen powder.

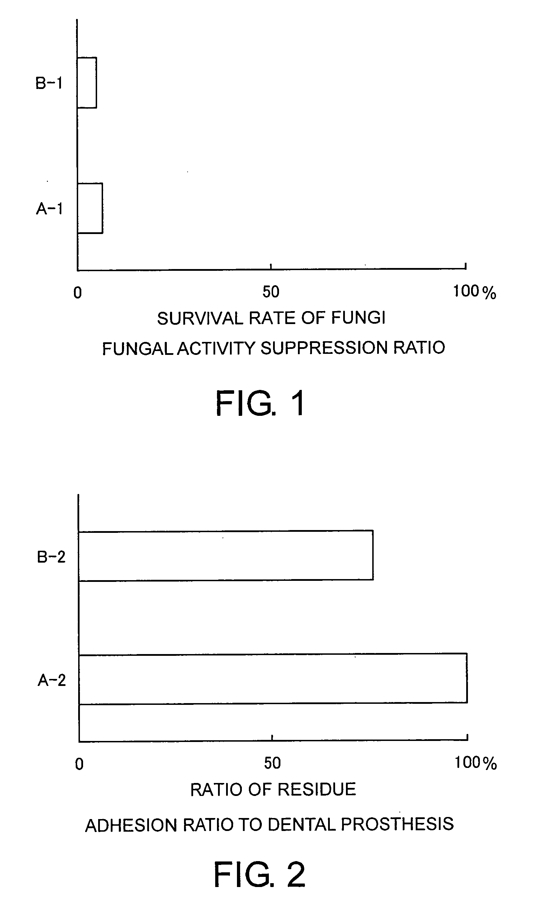

Referring to Fig. 1, the composition of this invention containing miconazole and collagen is represented by A-1. By immersing the composition A-1 into purified water, a fungal activity suppression ratio (anti-fungal activity) was measured.

With respect to 100 wt % of the purified water, 30 wt % of collagen and 1 wt % of miconazole were used.

A dental prosthesis was formed by resin. To the dental prosthesis, fungi as offending bacteria causing denture candidasis were adhered. The composition A-1 was attached onto the fungi adhered to the dental prosthesis. Thereafter, a plastic plate was put on the dental prosthesis. The dental prosthesis was immersed in the purified water and left untreated for 24 hours. Thereafter, the fungal activity suppression ratio was measured.

On the other hand, a comparative composition B-1 comprising miconazole alone was prepared. The fungal activity suppression ratio was similarly measured by immersing the comparative composition B-1 into purified water.

With respect to 100 wt % of the purified water, 1 wt % of miconazole was used.

A dental prosthesis was formed by resin. To the dental prosthesis, fungi as offending bacteria causing denture candidasis were adhered. The comparative composition B-1 was attached onto the fungi adhered to the dental prosthesis. Thereafter, a plastic plate was put on the dental prosthesis. The dental prosthesis was immersed in the purified water and left untreated for 24 hours. Thereafter, the fungal activity suppression ratio was measured.

As seen from Fig. 1, in case where the composition A-1 was used, about 6.5 % of the fungi survived after lapse of 24 hours. Therefore, the composition A-1 had the fungal activity suppression ratio of about 93.5 %. On the other hand, in case where the comparative composition B-1 was used, about 5.0 % of the fungi survived after lapse of 24 hours. Therefore, the comparative composition B-1 had the fungal activity suppression ratio of about 95.0 %.

From the above, it is obvious that the composition A-1 exhibits the fungal activity suppression ratio substantially equivalent to that of the comparative composition B-1 although the composition A-1 contains collagen.

Referring to Fig. 2, comparison in adhesion ratio to the dental prosthesis is made between a composition A-2 containing miconazole and collagen according to this invention and a comparative composition B-2.

By immersing the composition A-2 of this invention into purified water, an adhesion ratio was measured.

The composition A-2 of this invention comprises miconazole and collagen. With respect to 100 wt % of the purified water, 30 wt % of collagen and 1 wt % of miconazole were used.

A dental prosthesis was formed by resin. To the dental prosthesis, the composition A-2 was attached. Thereafter, a plastic plate was put on the dental prosthesis. The dental prosthesis was immersed in the purified water and left untreated for 24 hours. Thereafter, the adhesion ratio to the dental prosthesis was measured.

On the other hand, a comparative composition B-2 comprising miconazole alone was prepared. The adhesion ratio was similarly measured by immersing the comparative composition B-2 into purified water.

With respect to 100 wt % of the purified water, 1 wt % of miconazole was used.

A dental prosthesis was formed by resin. To the dental prosthesis, the comparative composition B-2 was attached . Thereafter, a plastic plate was put on the dental prosthesis. The dental prosthesis was immersed in the purified water and left untreated for 24 hours. Thereafter, the adhesion ratio to the dental prosthesis was measured.

Measurement of the adhesion ratio to the dental prosthesis was carried out in the following manner. The surface of the dental prosthesis and the plastic plate were stained to confirm the residue of each of the composition A-2 and the comparative composition B-2.

As seen from Fig. 2, the adhesion ratio of the comparative composition B-2 was about 76% assuming that the adhesion ratio of the composition A-2 is 100%.

Therefore, the adhesion ratio of the composition A-2 is substantially higher than that of the comparative composition B-2. This means that the composition according to this invention can stably be held on the dental prosthesis for a long time so as to exhibit a fungal activity suppression ratio substantially equivalent to the existing miconazole gel, even if the concentration of miconazole in the composition is lower than that of the existing miconazole gel.

In the foregoing examples, collagen was used as a specific example of the adhesive protein. However, this invention is not limited thereto but a different kind of protein may be used. For example, it has been confirmed that, when laminin or fibronectin is mixed with miconazole instead of collagen, the fungal activity suppression ratio and the adhesion ratio to the dental prosthesis were equivalent to those of collagen.

The composition of this invention can be used in the form of gel. If necessary, a known additive may be mixed with the effective components (i.e., miconazole and adhesive protein) of this invention by an ordinary technique to produce a drug for external use, such as liquid, lotion, emulsion, tinctura, ointment, cream, aqueous gel, oil-based gel, and aerozol.

As described above, the composition of this invention contains miconazole and adhesive protein. Therefore, the composition exhibits an excellent effect against denture candidasis at a lower concentration and without adverse effects as compared with an existing antifungal agent comprising miconazole gel.

Further, by the use of the composition of this invention, miconazole can be adhered to and held on the dental prosthesis for a long time by inclusion of the adhesive protein. It is therefore possible to increase the fungal activity suppression ratio for the fungi causing the denture candidasis in the oral cavity and to suppress the growth of the fungi.

While this invention has thus far been described in conjunction with the preferred embodiment thereof, it will readily be possible for those skilled in the art to put this invention into practice in various other manners.

## Claims

1. A composition for an antifungal agent, comprising miconazole, which is an imidazole antifungal agent, and adhesive protein.

2. A composition according to claim 1, wherein the adhesive protein is one selected from collagen, laminin and fibronectin.

3. A composition according to claim 1, or 2, wherein the adhesive protein is in the form of powder, the powder being mixed with the miconazole.

4. A composition according to claim 1, 2, or 3, wherein the composition is in the form of gel.
